# EUROPEAN PATENT APPLICATION

(11) **EP 2 801 922 A1**
(43) Date of publication of application: **12.11.2014**
(21) Application number: 13167341.0
(22) Date of filing: 10.05.2013
(51) Int. Cl.: G06F 19/28, G06Q 50/22

(54) **Genetic database system and method**

(71) Applicant: Alexandre, Laurent, 1050 Brussels (BE)
(72) Inventor: Alexandre, Laurent, 1050 Brussels (BE)
(74) Representative: Pronovem

(57) **Abstract**

A system and method for performing an analysis of a subject's genome, transcriptome and/or epigenome is described. The identities of a predetermined number of, such as ten or more, characteristics in the subject's genome, transcriptome and/or epigenome which are associated with one or more particular phenotype(s) or an increased risk of a particular phenotype are determined by performing an analytical technique upon a biological sample obtained from the subject. The amounts of any royalties or fees required for determination of the identities of one or more of the ten or more characteristics in the subject's genome, transcriptome and/or epigenome are determined via a computer. The amount of a fee charged for a determination of the identities of the ten or more characteristics in the subject's genome, transcriptome and/or epigenome are adjusted via a computer to reflect the amount of the determined royalties or fees and/or the amount of a fee for a future treatment of the subject. A database accessed during the method is also described.

## Description

### BACKGROUND

### Field of the Invention

The present invention is related to a method comprising steps and means that take into consideration the respect of industrial property rights in the sequencing and the genetic analysis of genome, transcriptome and epigenome specific of a subject, especially a human individual.

### Description of the Related Art

In recent years, DNA sequencing technologies and data-production pipelines technologies have provided remarkable improvements.

For many years, the cost-accounting data (that include costs per Megabase of DNA sequencing or the costs of determining one Megabase (a million bases) of DNA sequence of a specific quality) are following "the Moore's Law".

However, starting from the year 2007, costs do not follow the Moore's Law anymore and are decreasing much more quickly than expected. (www.genome.gov/sequencingcosts).

Therefore, these costs are not only related to the Moore's Law, which describes a long term trend in the computer hardware industry that involves the doubling of "computing power" every two years.

Indeed, until 2007, the DNA sequencing technology represents the costs of generating DNA sequence using Sanger-based chemistries and capillary-based instruments ("first generation" sequencing platforms).

Starting from 2005 DNA sequencing began using "second generation" of sequencing platforms, which allow to control or reduce heavily sequencing costs.

Therefore, the number of people that can be sequenced at a low cost is also increasing. This means that in a delay of few years, a large percentage of the human population, especially in developed countries will be sequenced, which means that genetic modifications in their genome or epigenome can be more quickly identified.

This will lead to a new paradigm in human medicine that may improve diagnosis, treatment and/or prevention of several diseases and syndromes, especially genetic related diseases and syndromes.

Until now, it is not clear which national or international Authority will collect all related information in order to keep confidentiality upon the detected genetic modifications in a specific genome, transcriptome or epigenome of a sequenced subject, especially a human individual.

In our Economy, there is also a need to respect industrial property rights protecting research dedicated to the detection of specific genetic modification(s) present in the genome, transcriptome or the epigenome of one or more specific subject(s) or population.

Therefore, there is a need in some cases to develop a new and efficient method comprising steps and means that are related to the sequencing of a subject genome or epigenome and that take into consideration the respect of industrial property rights and their costs.

### SUMMARY

The present invention is related to a method for analyzing a subject's genome to assess whether the subject suffers from or is susceptible to suffer from one or more genetic related disease(s) or disorder(s). In some embodiments one subject is a specific mammal species, preferably a human individual (including a human patient that could also correspond to a human embryo or a human fetus), which preferably comprises an Electronic Medical Record (EMR).

The present invention is also related to a method for performing an analysis of a subject's genome, the method comprising determining the identities of one or more, preferably two or more, more preferably ten or more characteristics in the subject's genome, transcriptome and/or epigenome which are associated with one or more particular phenotype(s) or with an increased risk of a particular phenotype by performing an analytical technique upon a biological sample obtained from the subject; determining (via a computing device or computer) the amounts of any royalties or fees required for determination of the identities of one or more (two or more, preferably of the ten or more) characteristics in the subject's genome and/or epigenome; and adjusting (via a computing device or computer) the amount of a fee charged for a determination of the identities of the one or more (two or more, preferably ten or more) characteristics in the subject's genome, transcriptome and/or epigenome, to reflect the amount of the determined royalties or fees and/or the amount of a fee for the future treatment of the subject.

According to the invention, the one or more, preferably two or more , more preferably ten or more) characteristics may be selected from the group consisting of an allele of a gene, a polymorphic nucleotide, a deletion of one or more nucleotides, an insertion of one or more nucleotides, a duplication or triplication of one or more nucleotides, a translocation of one or more nucleotides, an inversion of one or more nucleotides, a nucleotide copy number variation, an ( transcript) expression level of a nucleic acid sequence or portion thereof, a methylation status of one or more nucleotides, or any other genomic modification, including multiple nucleotide polymorphisms (MNP).

Preferably, in the present invention, the particular phenotype is a genetic related disorder or disease affecting the subject or susceptible to affect the subject.

In the present invention, the one or more, preferably the two or more, preferably ten or more characteristics of the subject's genome, transcriptome or epigenome can be more than 15, 20, 30, 40, 50, 100, 500 or more specific characteristics.

Determining the amounts of any royalties or fees required for determination of the identities of one or more (two or more, preferably ten or more) characteristics may comprise instructing a computer to access a database having stored therein information relating to a plurality of intellectual property documents covering the analysis of characteristics in a genome and/or epigenome; instructing a computer to determine whether the analysis of one or more of the ten or more characteristics in the subject's genome and/or epigenome is covered by any of the intellectual property documents; and if the analysis of one or more (two or more, preferably ten or more) characteristics in the subject's genome, transcriptome and/or epigenome is covered by the intellectual property, instructing a computer to determine the amount of the royalties or fees due for the analysis of one or more (two or more, preferably ten or more) characteristics in the subject's genome and/or epigenome. The intellectual property documents may be patents.

The database may comprise information relating to the characteristics covered by each of the patents, the countries in which each of the patents are issued, and the royalty or fee required under each of the patents in each of the countries. Determining whether the analysis of one or more (two or more, preferably ten or more) characteristics in the subject's genome, transcriptome and/or epigenome is covered by the intellectual property may comprise comparing each of the one or more (two or more , preferably ten or more) characteristics analyzed in the subject's genome, transcriptome and/or epigenome to the characteristics covered by each of the patents in the database which have issued in the country where the analytical technique was performed on the biological sample to identify any of the one or more (two or more, preferably ten or more) characteristics which are covered by intellectual property in the country.

The database may further comprise the expiration dates of each of the patents in each of the countries. Determining whether the analysis of one or more (two or more , preferably ten or more) characteristics in the subject's genome, transcriptome and/or epigenome is covered by the intellectual property further may comprise identifying any of the patents issued in the country where the analytical technique was performed on the biological sample which had not expired on the date on which the analytical technique was performed.

The database may further comprise information for each patent relating to whether a royalty or fee is due under the patent if the subject is negative for the characteristic described therein, whether a royalty or fee is due under the patent if the subject is positive for the characteristic described therein, whether a royalty or fee is due under the patent if the subject is either positive or negative for the characteristic described therein, whether a royalty or fee is due only if the characteristic has a specific identity or whether a royalty or fee is due regardless of the identity of the characteristic. Determining whether the analysis of one or more (two or more, preferably the ten or more) characteristics in the subject's genome, transcriptome and/or epigenome is covered by the intellectual property may further comprise comparing the status of each of the one or more (two or more, preferably ten or more) characteristics in the subject to the statuses of the characteristics which are royalty or fee bearing under each of the patents.

The subject may be a mammal species, preferably a human individual (including a human patient, a human embryo or human fetus). The method may additionally comprise recording the results obtained from the analytical technique in an electronic medical record (EMR) of the subject.

In another embodiment, there is a method for performing an analysis of a subject's genome, transcriptome and/or epigenome, the method comprising determining the identities of one or more (two or more, preferably ten or more) characteristics in said subject's genome, transcriptome and/or epigenome which are associated with one or more particular phenotype(s) or with an increased risk of one or more particular phenotype(s) by performing an analytical technique upon a biological sample obtained from said subject; determining, via a computer, the amounts of any royalties or fees required for determination of the identities of one or more of said one or more (two or more, preferably ten or more) characteristics in the subject's genome, transcriptome and/or epigenome; and adjusting (via a computing device or computer) the amount of a fee charged for a determination of the said identities of said one or more (two or more , more preferably ten or more) characteristics in the subject's genome, transcriptome and/or epigenome, to reflect the amount of the determined royalties or fees and/or the amount of a fee for the future treatment of said subject.

In another embodiment, there is a computerized system for performing an analysis of a subject's genome, transcriptome and/or epigenome, the system comprising a database configured to store information regarding the identities of one or more two or more, preferably ten or more) characteristic(s) in the subject's genome, transcriptome and/or epigenome which are associated with particular phenotypes or with an increased risk of particular phenotypes and information regarding the amounts of any royalties required for determination of the identities of one or more of said one or more (ten or more) characteristic(s) in the subject's genome, transcriptome and/or epigenome; and a processor configured to perform instructions for determining the amount of any royalties required for determination of the identities of one or more of said one or more (ten or more) characteristics in the subject's genome, transcriptome and/or epigenome, and adjusting the amount of a fee charged for said determination of said identities of said one or more (ten or more) characteristics in the subject's genome, transcriptome and/or epigenome to reflect the amount of the determined royalties.

In another embodiment, there is a non-transitory computer readable medium containing program instructions for genetic analysis, wherein execution of the program instructions by a computing environment carries out a method, comprising retrieving information regarding identities of one or more, (preferably two or more, more preferably ten or more) characteristic(s) in a subject's genome, transcriptome and/or epigenome which are associated with particular phenotypes from a database, retrieving information regarding amounts of any royalties required for determination of said identities of one or more of said (preferably two or more; ten or more) characteristic(s) in the subject's genome, transcriptome and/or epigenome from a database, determining amounts of any royalties required for determination of the identities of one or more of said (preferably two or more, more preferably ten or more) characteristic(s) in the subject's genome, transcriptome and/or epigenome, and adjusting the amount of a fee charged for said determination of said identities of said (preferably two or more , more preferably ten or more) characteristic(s) in the subject's genome, transcriptome and/or epigenome to reflect the amount of the determined royalties.

In another embodiment, there is a method to identify genetic modifications in a specific subject suffering or susceptible to suffer from one or more genetic related disorder(s) or disease(s) affecting this specific subject, the method comprising performing a complete, partial or targeted sequencing of the genome, transcriptome or the epigenome of a biological sample obtained from said specific subject; obtaining genetic analysis of genome, transcriptome or epigenome of the biological sample by comparing every genetic modification present in the said sample genome, said sample transcriptome or said sample epigenome with (more than ten) specific genome, transcriptome and/or epigenome characteristic(s) associated one or more genetic related disorder(s) or disease(s) affecting or susceptible to affect a subject, the said (ten or more) specific genome, transcriptome and/or epigenome characteristic(s) being present in a centralized database; identifying at least one genome, transcriptome and/or epigenome characteristic specific of the status of at least one of the said disorders or diseases or of the evolution of the said disorders or diseases; collecting positive results obtained from said comparison in a database specific for each subject genome, transcriptome and/or epigenome sequenced; and adapting the cost or fee of the analysis of this sequencing or adapting the costs or fees of a future treatment of the said subject, according to at least one royalty rate of at least one property right protecting the identification of one or more of the said specific genome, transcriptome and/or epigenome characteristic(s).

The genetic analysis may be obtained by comparing every genetic modification present in the sample genome, transcriptome or epigenome with one or more, preferably at least 10,15, 20, 30, 40, 50 or more specific genome, transcriptome and/or epigenome characteristics associated with at least one genetic related disorder(s) or disease(s) affecting the subject. The genetic analysis may be obtained by comparing every genetic modification present in the sample genome, transcriptome or epigenome with 100 or more specific genome, transcriptome and/or epigenome characteristics associated with at least one genetic related disorder(s) or disease(s) affecting the subject. The genetic analysis may be obtained by comparing every genetic modification present in the sample genome or epigenome with 500 or more specific genome, transcriptome and/or epigenome characteristics associated with at least one genetic related disorder(s) or disease(s) affecting the subject. The subject may be a human individual, including a human embryo or human fetus. The method may additionally comprise recording positive results obtained from the analysis of the sequencing upon the subject's electronic medical record (EMR).

This genetic analysis may be also obtained by comparing every genetic modifications present in the sample genome, transcriptome or epigenome with a reference genome, such as the human reference genome, transcriptome or epigenome sequence, preferably the one identified by the Genome. Reference Consortium (GRC), now released by the reference GRCh37, also called hgI9, derived from anonymous volunteers or from numerous genomes regions presenting high or low allelic diversity and obtainable from public databases, such as Ensemble or UCSG Genome Browser.

The genome and/or epigenome characteristic may be a benign or malign cancer genome, transcriptome and/or epigenome characteristic. The biological sample may be any biological fluid obtained from the tested subject, preferably a human patient, such as blood, urine, cerebrospinal fluid, saliva, ...or a cell sample or a tissue sample obtained from a tumoral tissue of the subject and possibly from another non-tumoral tissue of the same subject.

The genome, transcriptome and/or epigenome characteristic may be a diabetes genome, transcriptome and/or epigenome characteristic. The genome, transcriptome and/or epigenome characteristic may be an auto-immune genome, transcriptome and/or epigenome characteristic. The genome, transcriptome and/or epigenome characteristic may be a hypertension genome, transcriptome and/or epigenome characteristic. The genome, transcriptome and/or epigenome characteristic may be an obesity genome, transcriptome and/or epigenome characteristic. The genome, transcriptome and/or epigenome characteristic may be a heart disease genome, transcriptome and/or epigenome characteristic.

The genome, transcriptome and/or epigenome characteristic may be a neurological genome, transcriptome and/or epigenome characteristic. The neurological disease may be selected from the group consisting of Alzheimer's disease, Parkinson's disease and/or Huntington's disease.

The specific genome, transcriptome and/or epigenome characteristic may be selected from the group consisting of Angelman syndrome, Canavan disease, Coeliac disease, Charcot-Marie-Tooth disease, Color blindness disease, Cri du chat disease, Cystic fibrosis disease, Down syndrome, Duchenne muscular dystrophy, Haemochromatosis, Haemophilia, Klinefelter's syndrome, Neurofibromatosis, Phenylketonuria, Polycystic kidney disease, Prader-Willi syndrome, Sickle-cell disease, Tay-Sachs disease, Turner syndrome or any other genetic related syndrome or disease not yet identified.

The biological sample may be a germinal cell or a mixture of germinal cells. The biological sample may be a somatic cell or a mixture of somatic cells. The method may additionally comprise the step of identifying the sex of the subject (but preferably not made upon an human embryo or human fetus). The method may be repeated upon the same specific subject at an interval time of a few days, a few weeks, a few months preferably an interval time comprised between about one year and about ten years.

The complete, partial or targeted sequencing may be done upon any nucleic acid sequence ((circulating) DNA or RNA sequences) and may be based upon methods selected from the group consisting of genetic, transcriptogenomic or epigenenomic analyses, SNP genotyping, detection of sequence methylation, mapping, sequencing, use of high density or low density microarrays, pyrosequencing, gene expression analysis or quantitative genetic amplification and/or DNA fingerprinting. Circulating DNA present in blood of a tested subject and being released by cells following their possible lysis is a good example of sample analysis, not made upon cells. The comparing step may be performed upon multigenic variation, genetic modification or monogenic genome, transcriptome and/or epigenome characteristic genetic modification; these modifications being identified in affected cells (such as tumoral cells) and being compared to normal healthy cells (i.e. obtained from subjects being not affected by any of genetic diseases or syndromes to be detected).. The method may further comprise issuing an invoice reflecting the adapted cost.

In yet another embodiment, there is a method for performing an analysis of a subject's genome, transcriptome or epigenome, the method comprising determining the identities of (preferably two or more, more preferably ten or more) characteristic(s) in said subject's genome, transcriptome and/or epigenome which are associated with one or more particular phenotype(s) or with an increased risk of one or more particular phenotype(s), by performing an analytical technique upon a biological sample obtained from said subject, wherein said analytical technique is able to determine identities of the said (preferably two or more, more preferably at least ten or more) characteristic(s) of the subject's genome and/or epigenome; determining the amounts of any royalties required for determination of the identities of one or more of said (preferably two or more, more preferably ten or more) characteristic(s) in the subject's genome, transcriptome and/or epigenome; and adjusting the amount of fee charged for a determination of the said identities of said (preferably two or more , more preferably ten or more) characteristics in the subject's genome, transcriptome and/or epigenome, to reflect the amount of the determined royalties and/or the amount of fee for the future treatment of said subject. The present invention will be described in the following examples in reference to the following schematic figures presented as preferred embodiments that do not limit the scope of the present invention.

### BRIEF DESCRIPTION OF THE DRAWINGS

Figure 1A is a diagram of an example of one embodiment of a configuration for operating a system and method for conducting a genetic analysis.

Figure 1B is a diagram of an example of another embodiment of a configuration for operating a system and method for conducting a genetic analysis.

Figure 2 is a flow diagram of an example of one embodiment of a method operating on the system shown in Figure 1A or Figure 1B.

Figure 3 is a flow diagram of an example of an embodiment of a method function or a module to ascertain royalties or fees as shown in Figure 2.

Figure 4 is a flow diagram of an example of an embodiment of a method function or a module to determine whether analysis of characteristics is covered by intellectual property (IP) as shown in Figure 3.

Figure 5 is a flow diagram of another example of an embodiment of a method function or a module to determine whether analysis of characteristics is covered by IP as shown in Figure 3.

Figure 6 is a flow diagram of yet another example of an embodiment of a method function or a module to determine whether analysis of characteristics is covered by IP as shown in Figure 3.

Figure 7 is an example of an embodiment of a database accessed during the method of Figure 2.

Figure 8 is a flow diagram of an example of an embodiment of a method to build a genetic database such as shown in Figure 7.

Figure 9 is a flow diagram of another example of an embodiment of a method operating on the system shown in Figures 1A or 1B.

Figure 10 is a flow diagram of an example of an embodiment of a method function or a module to obtain a genetic analysis as shown in Figure 9.The drawings

### DETAILED DESCRIPTION OF CERTAIN EMBODIMENTS

The following detailed description of certain embodiments presents various descriptions of specific embodiments of the invention. However, the invention can be embodied in a multitude of different ways as defined and covered by the claims. In this description, reference is made to the drawings wherein like parts are designated with like numerals throughout.

The terminology used in the description presented herein is not intended to be interpreted in any limited or restrictive manner, simply because it is being utilized in conjunction with a detailed description of certain specific embodiments of the invention. Furthermore, embodiments of the invention may include several novel features, no single one of which is solely responsible for its desirable attributes or which is essential to practicing the inventions herein described. Unless specifically defined herein, all terms used herein have the same meaning as they would to one skilled in the art of the present invention. Practitioners are particularly directed to Sambrook et al, molecular Cloning A laboratory Manual , 4th Ed, Cold Spring Harbor Press, Plainsview , New York (2012) and Ausubel et al, current Protocols in Molecular Biology ( Supplement 100) John Wiley & Sons, New York (2012) for definitions and terms of the art.

The system and method described herein can be implemented on various configurations of hardware and software. The system can be comprised of various modules, tools, and applications as discussed below. As can be appreciated by one of ordinary skill in the art, each of the modules may comprise various sub-routines, procedures, definitional statements and macros. Each of the modules are typically separately compiled and linked into a single executable program. Therefore, the following description of each of the modules is used for convenience to describe the functionality of the preferred system. Thus, the processes that are undergone by each of the modules may be arbitrarily redistributed to one of the other modules, combined together in a single module, or made available in, for example, a shareable dynamic link library. Depending on the embodiment, certain modules may be removed, merged together, or rearranged in order. Also depending on the embodiment, certain steps of the methods may be added, rearranged, combined, or removed.

The system modules, tools, and applications may be written in any programming language such as, but not restricted to C, C++, C#, BASIC, Visual Basic, Pascal, Ada, Java, HTML, XML, or FORTRAN, and executed on an operating system, such as variants of Windows, Macintosh, UNIX, Linux, VxWorks, or other operating system. C, C++, C#, BASIC, Visual Basic, Pascal, Ada, Java, HTML, XML and FORTRAN are industry standard programming languages for which many commercial compilers can be used to create executable code.

### Overview

In certain embodiments there is a method to identify genetic modifications in a specific subject suffering or susceptible to suffer from one or more genetic related disease(s) or disorder(s) susceptible to affect this specific subject, especially a subject being a specific human individual.

In certain embodiments, the method can comprise at least the steps of performing complete, partial or targeted sequencing of the genome, transcriptome or the epigenome of a biological sample obtained from a specific subject; obtaining genetic analysis of genome, transcriptome or epigenome of the biological sample by comparing every genetic modification present in this sample genome, this sample transcriptome or this sample epigenome with more than 10, 20, 30, 40, 50, 100, 500 or more specific genome, transcriptome and/or epigenome characteristics associated with at least one genetic related disorder or disease affecting or susceptible to affect this subject, these more than 10, 20, 30, 40, 50, 100, 500 or more specific genome, transcriptome and/or epigenome characteristics being stored in a centralized database; identifying at least one genome, transcriptome and/or epigenome characteristic specific of the status of at least one of these disorders or diseases or of the evolution of these disorders or diseases; collecting positive results obtained from this comparison step in a database specific to each subject genome, transcriptome and/or epigenome sequence; and adapting a cost or fee of the analysis of this sequencing or adapting the costs of future treatment of this subject, according to the identified property rights. The property rights can include at least one royalty rate of property right, preferably a patent, protecting the identification of these specific genome and/or epigenome characteristics.

The subject can be any individual animal species, preferably a mammal species, more preferably a human individual (including a human patient, a human embryo or human fetus), which preferably comprises a corresponding Electronic Medical Record (EMR).

Indeed, the method may comprise advantageously the step of recording the positive results obtained from the analysis of this sequencing upon an Electronic Medical Record (EMR) of the tested subject.

According to the method, the genome, transcriptome and/or epigenome characteristics can be a cancer genome, transcriptome and/or epigenome characteristics specific of cancer or tumoral diseases or hyper proliferative disorders. These characteristics could correspond to a benign or malignant cancer disorder of the subject or a disorder susceptible to affect the subject in the future (in a delay of about a few weeks, about a few months, about one year, about two years, about three years, about four years, about five years, about six years, about seven years, about nine years, about ten years, about fifteen years, about twenty years, about twenty five years, about thirty years, about thirty five years, about forty years, about forty five years, about fifty years, about sixty years, about seventy years, about seventy five years or more).

If the genome, transcriptome and/or epigenome characteristic is a cancer characteristic, the biological sample is preferably a cell sample or a tissue sample obtained from a tumoral tissue of the subject, possibly compared to another non-tumoral tissue from the same subject (which means another tissue of the same subject, but not affected by the tumor disease or disorder).

In the method, the genome, transcriptome and/or epigenome characteristic can also be a characteristic associated with or associated with an increased risk of a condition selected from the group consisting of diabetes, auto-immune conditions, hypertension, obesity, nerve disease or neurological conditions such as Alzheimer's disease, Parkinson's disease and/or Huntington's disease.

In the method, this characteristic can also be associated with or associated with an increased risk of a condition selected from the group consisting of Angelman syndrome, Canavan disease, Coeliac disease, Charcot-Marie-Tooth disease, Color blindness disease, Cri du chat disease, Cystic fibrosis disease, Down syndrome, Duchenne muscular dystrophy, Haemochromatosis, Haemophilia, Klinefelter's syndrome, Neurofibromatosis, Phenylketonuria, Polycystic kidney disease, Prader-Willi syndrome, Sickle-cell disease, Tay-Sachs disease, Turner syndrome or cancer (or tumor being equivalent term to cancer).

In the method, the biological sample can also be a germinal cell or a mixture of germinal cells, but preferably can be a somatic cell or a mixture of somatic cells and may also comprise the step of an identification of the sex of the subject (but preferably not upon an human embryo or human fetus).

In certain embodiments, the different steps of the method can be repeated upon the same specific subject at an interval time of about a few days, preferably a few weeks, a few months, more preferably an interval time comprised between about one year and about ten years, preferably between about two years and about five years.

Specific steps of the method can be obtained by methods and means well known by the person skilled in the art to allow a complete partial or targeted sequencing of different genetic elements, such as the genome, transcriptome or the epigenome of a biological sample comprising a genetic material through known techniques, preferably selected from the group consisting of genetic, transcriptogenomic or epigenomic analyses such as SNP genotyping, detection of sequence methylation, mapping, sequencing, use of high density or low density microarrays, pyrosequencing, gene expression analysis or quantitative genetic amplification and/or DNA fingerprinting.

A comparing step of the method could be performed upon a multigenic variation, multi-epigenic variation, genetic modification or monogenic genome, transcriptome and/or epigenome characteristic genetic modification.

The method can also comprise the step of issuing an invoice reflecting the adapted cost obtained by the method.

Another aspect concerns a method for performing an analysis of a subject's genome, transcriptome or epigenome comprising determining the identities of ten or more characteristics in this subject's genome, transcriptome and/or epigenome which are possibly associated with at least one particular phenotype, by performing an analytical technique upon a biological sample obtained from the subject, wherein this analytical technique is able to determine identities of these ten or more characteristics of the subject's genome, transcriptome and/or epigenome; determining the amounts of any royalties required for determination of the identities of one or more of these ten or more characteristics in the subject's genome, transcriptome and/or epigenome; and adjusting the amount of a fee charged for a determination of these identities of these ten or more characteristics in the subject's genome, transcriptome and/or epigenome, to reflect the amount of the determined royalties and/or the amount of fee for the future treatment of the subject.

Another aspect concerns a (computerized) system for performing an analysis of a subject's genome, transcriptome and/or epigenome comprising a memory or a database to store information regarding the identities of ten or more characteristics in the subject's genome, transcriptome and/or epigenome which are possibly associated with particular phenotypes and information regarding the amounts of any royalties required for determination of the identities of one or more of these ten or more characteristics in the subject's genome, transcriptome and/or epigenome; and a processor to perform the steps of determining the amounts of any royalties required for determination of the identities of one or more of these ten or more characteristics in the subject's genome, transcriptome and/or epigenome; and adjusting the amount of a fee charged for the determination of the identities of these ten or more characteristics in the subject's genome, transcriptome and/or epigenome to reflect the amount of the determined royalties.

Another aspect is related to a computer readable medium comprising instructions to retrieve information regarding the identities of ten or more characteristics in a subject's genome, transcriptome and/or epigenome which are associated with particular phenotypes from a database; retrieve information regarding the amounts of any royalties required for determination of these identities of one or more these ten or more characteristics in the subject's genome, transcriptome and/or epigenome from a database; determine the amounts of any royalties required for determination of the identities of one or more of these ten or more characteristics in the subject's genome, transcriptome and/or epigenome; and adjust the amount of a fee charged for this determination of these identities of these ten or more characteristics in the subject's genome, transcriptome and/or epigenome, to reflect the amount of the determined royalties.

In other embodiments, the ten or more characteristics can be a different number, such as being in a range from (about) 2 characteristics up to (about) 500 characteristics (inclusive of each integer therebetween) or more than 500 characteristics

### Definitions

The following provides a number of useful possible definitions of terms used in describing certain embodiments of the disclosed development. As used herein, the articles "a" and "an" are used herein to refer to one or to more than one (i.e. to at least one) of the grammatical object of the article. "about" as used herein when referring to a measurable value such as an amount, temporal duration, and the like is meant to encompass variations of 20% or 10%, more preferably 5% or lower% from the specified value, as such variations are appropriate to perform the disclosed methods.

A network may refer to a network or combination of networks spanning any geographical area, such as a local area network (LAN), wide area network (WAN), regional network, national network, and/or global network. The Internet is an example of a current global computer network. Those terms may refer to hardwire networks, wireless networks, or a combination of hardwire and wireless networks. Hardwire networks may include, but are restricted to fiber optic lines, cable lines, ISDN lines, copper lines, etc. Wireless networks may include, for example, cellular systems, personal communications service (PCS) systems, satellite communication systems, packet radio systems, and mobile broadband systems. A cellular system may use, for example, code division multiple access (CDMA), time division multiple access (TDMA), personal digital phone (PDC), Global System Mobile (GSM), or frequency division multiple access (FDMA), among others.

A website may refer to one or more interrelated web page files and other files and programs on one or more web servers. The files and programs are accessible over a computer network, such as the Internet, by sending a hypertext transfer protocol (HTTP or HTTPS [S-HTTP]) request specifying a uniform resource locator (URL) that identifies the location of one of the web page files, where the files and programs are owned, managed or authorized by a single business entity. Such files and programs can include, but are not restricted to, hypertext markup language (HTML) files, common gateway interface (CGI) files, and Java applications. The web page files preferably include a home page file that corresponds to a home page of the website. The home page can serve as a gateway or access point to the remaining files and programs contained within the website. In one embodiment, all of the files and programs are located under, and accessible within, the same network domain as the home page file. Alternatively, the files and programs can be located and accessible through several different network domains.

A web page or electronic page may include that which is presented by a standard web browser in response to an HTTP request specifying the URL by which the web page file is identified. A web page can include, for example, text, images, sound, video, and animation.

A computer or computing device may be any processor controlled device. The computer or computing device may be a device that permits access to the Internet, including terminal devices, such as personal computers, workstations, servers, clients, mini-computers, main-frame computers, laptop computers, a network of individual computers, mobile computers, palm-top computers, hand-held computers, set top boxes for a television, other types of web-enabled televisions, interactive kiosks, personal digital assistants (PDAs), interactive or web-enabled wireless communications devices, mobile web browsers such as operating on a smartphone, or a combination thereof. The computer(s) or computing device(s) may further possess one or more input devices such as a keyboard, mouse, touch pad, joystick, pen-input-pad, and the like. The computer(s) or computing device(s) may also possess an output device, such as a visual display and an audio output. One or more of these computer(s) or computing devices may form a computing environment.

These computer(s) or computing device(s) may be uni-processor or multi-processor machine(s). Additionally, these computer(s) or computing device(s) may include an addressable storage medium or computer accessible medium, such as random access memory (RAM), an electronically erasable programmable read-only memory (EEPROM), programmable read-only memory (PROM), erasable programmable read-only memory (EPROM), hard disks, floppy disks, laser disk players, digital video devices, compact disks, video tapes, audio tapes, magnetic recording tracks, electronic networks, and any other techniques to transmit or store electronic content such as, by way of example, programs and data. In one embodiment, the computer(s) or computing device(s) are equipped with a network communication device such as a network interface card, a modem, or other network connection device suitable for connecting to the communication network. Furthermore, the computer(s) or computing device(s) execute an appropriate operating system such as Linux, UNIX, any of the versions of Microsoft Windows, Apple MacOS, IBM OS/2 or other operating system. The appropriate operating system may include a communications protocol implementation that handles all incoming and outgoing message traffic passed over the network. In other embodiments, while the operating system may differ depending on the type of computer or computing device, the operating system will continue to provide the appropriate communications protocols to establish communication links with the network.

The computer(s) or computing device(s) may contain program logic, or other substrate configuration representing data and instructions, which cause the computer to operate in a specific and predefined manner, as described herein. In one embodiment, the program logic may be implemented as one or more object frameworks or modules. These modules may be configured to reside on the addressable storage medium and configured to execute on one or more processors. The modules include, but are not limited to, software or hardware components that perform certain tasks. Thus, a module may include, by way of example, components, such as, software components, object-oriented software components, class components and task components, processes, functions, attributes, procedures, subroutines, segments of program code, drivers, firmware, microcode, circuitry, data, databases, data structures, tables, arrays, and variables.

The various components of the system may communicate with each other and other components comprising the respective computer(s) or computing device(s) through mechanisms such as, by way of example, interprocess communication, remote procedure call, distributed object interfaces, and other various program interfaces. Furthermore, the functionality provided for in the components, modules, and databases may be combined into fewer components, modules, or databases or further separated into additional components, modules, or databases. Additionally, the components, modules, and databases may be implemented to execute on one or more computer(s) or computing device(s). In another embodiment, some of the components, modules, and databases may be implemented to execute on one or more computers external to a website. In one instance, the website includes program logic, which enables the website to communicate with the externally implemented components, modules, and databases to perform the functions such as disclosed herein.

### Example Computing Environments

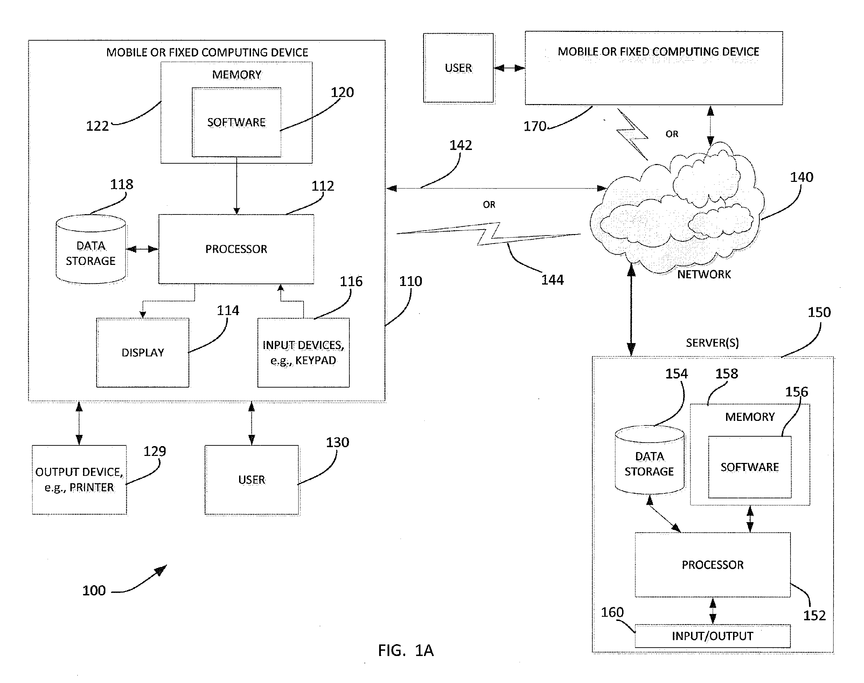

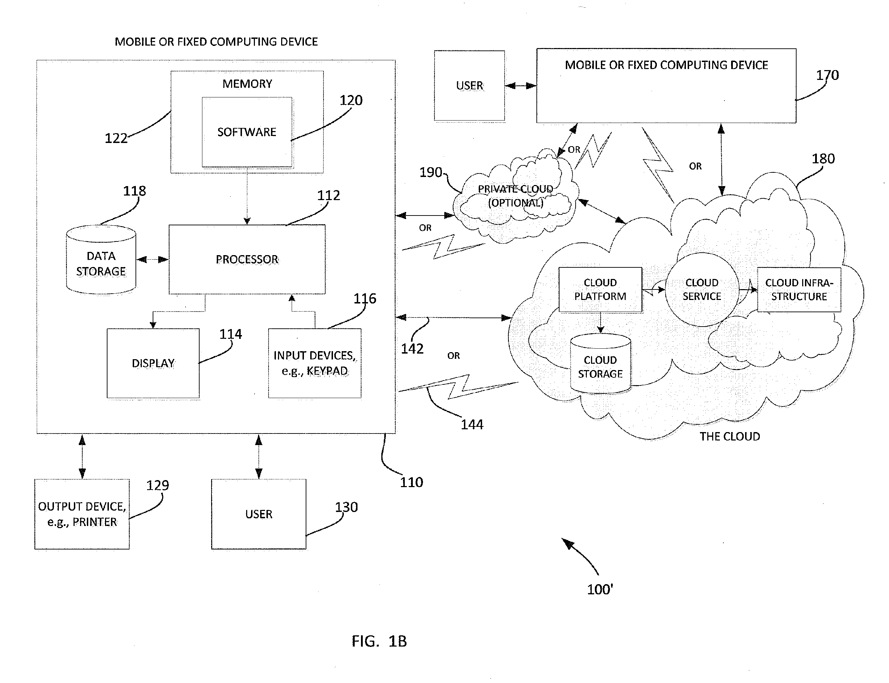

Certain embodiments of a system utilize a network as described in conjunction with Figure 1A herein below, or utilize a cloud, as described in conjunction with Figure 1B herein below. Certain embodiments are based on an example open system integrated architecture such as shown in Figure 1A and Figure 1B. In Figures 1A and 1B, the example open system integrated architecture may be based on, for example, a user interface interacting with a local or remote data repository and a local or remote application running on a local or remote application server, such as an application server 150. Figures 1A and 1B are block diagrams of an example system 100 that may be used to implement certain systems and methods described herein. The functionality provided for in the components and modules of computing system 100 may be combined into fewer components and modules or further separated into additional components and modules. Various other types of electronic devices communicating in a networked environment may also be used.

Referring to Figure 1A, an example configuration of components of an embodiment of the system 100 will now be described. A mobile or fixed computing device 110 is operated by a user 130. There may be other mobile or fixed computing device(s) or computer(s) such as a device 170 operated by other users. The computing device or computer 110 of the system according to the invention can be a handheld computing device or other portable computing device such as a Palm, Pocket personal computer (PC), Linux based handheld, PDA, smartphone such as an iPhone® or Android™ based phone, a tablet computer such as an iPad® or Android based tablet, or a PC having a display. In other embodiments, the computing device or computer of the system according to the invention can be any form of a network or Internet connected device, including but not limited to PCs, mobile devices, PDA, laptops, tablets, chips, keyboards, voice audio and video software, mouse, keypads, touch pads, track ball, microphones, videos, storage devices, network devices, databases, scanners, copiers, digital pens, image recognition software and device, screens and other forms of displays, netbooks and other forms of computer hardware. The computing device or computer 110 in certain embodiments can operate in a stand-alone (independent) manner. In other embodiments, the computing device or computer 110 is in communication with one or more servers 150 via a network 140, such as a local area network, a wide area network, or the Internet. The server(s) can include one or processors 152, memory 158, data storage 154 and system software 156 executed by the processor(s), and input or output devices 160. In certain embodiments, the data storage 154 stores one or more databases used by the system. The processor(s) 152 are in communication with the database(s) via a database interface, such as structured query language (SQL) or open database connectivity (ODBC). In certain embodiments, the data storage 154 is not included in server(s) 150, but is in data communication with the server(s) via the database interface. The connection from the computing device or computer 110 to the network 140 can be a wireless or a satellite connection 144 or a wired or direct connection 142. In certain embodiments, the server(s) are part of a web site, such as on an intranet or the Internet.

When the computing device or computer 110 is connected with the server(s) 150, the web site may optionally provide updates on new features. In another embodiment, the computing device or computer runs software for the system and method described herein only when connected to the server(s) 150.

The computing device or computer 110 can include a processor 112, memory 122, a display 114, and one or more input devices 116. The processor 112 can be in data communication with a data storage 118. In certain embodiments, the data storage 118 may store prior records of the user and/or other data or software. System software 120 can be executed by the processor 112. The system software 120 may include an application graphical user interface (GUI). The application GUI can include a database interface to the data storage 118 of the computing device. In certain embodiments, the software is loaded from the data storage 118. In embodiments where the computing device or computer 110 communicates with a web site, the processor utilizes browser software in place of or in addition to the software 120. The network browser may be, for example, Microsoft Internet Explorer®, Apple Safari®, Mozilla Firefox®, Google Chrome™, browsers from Opera Software™, and so forth. An optional output device 129, such as a printer is connected to the computing device 110.

Referring to Figure 1B, an example configuration of components of an embodiment of the system 100 using a cloud computing architecture will now be described. The configuration of components in Figure 1B is similar to that of Figure 1A except that the network 140 and servers 150 of Figure 1A are replace by the cloud 180 of Figure 1B. An optional private cloud 190 can also be utilized. Cloud computing can include web-based tools or applications that users can access and use through a web browser as if it were a program installed locally on their own computer. In certain embodiments, the cloud 180 can comprise various computers, servers and data storage devices that function to provide a cloud platform (e.g., a web front end), cloud service (e.g., a queue), cloud infrastructure, and cloud storage (e.g., a database). A public/external cloud can be used with a private cloud in a hybrid cloud or a combined cloud environment in certain embodiments

### Operation

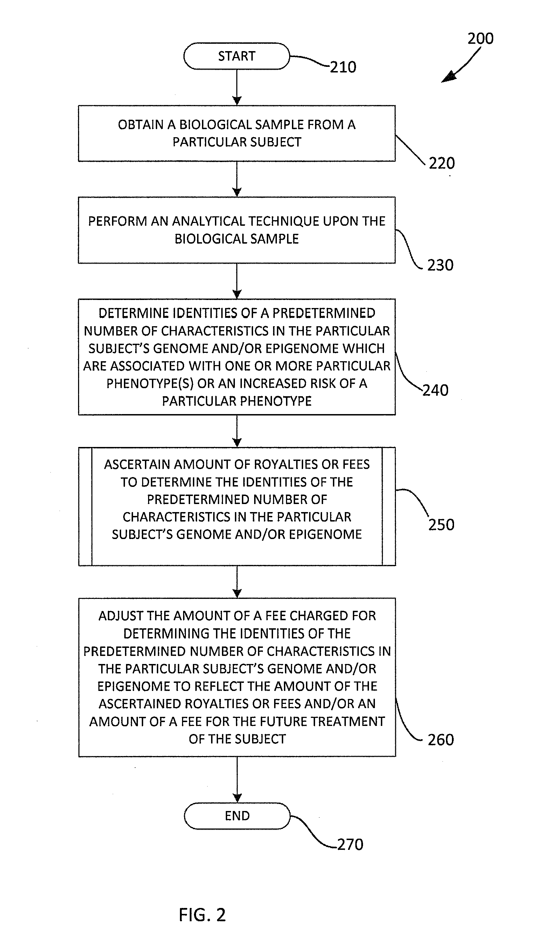

Referring to Figure 2, a top-level method 200 or a module will be described. Computer implemented steps of the method may be performed on the system 100 shown in Figure 1A or system 100' shown in Figure 1B. Depending on the embodiment, certain steps of the method may be added, rearranged, combined, or removed. Beginning at a start state 210, method 200 proceeds to a state 220 to obtain a biological sample from a particular subject. It will be appreciated that this step of the method may be performed without using a computer. The subject can be any individual animal species, preferably a mammal species, and more preferably a human individual including a human patient. In certain embodiments, the biological sample, can be a biological fluid collected from a mammal subject, including a human, preferably a biological fluid, such as blood, urine, cerebrospinal fluid, saliva,... or is a cell sample or a tissue sample obtained from a tumoral tissue of the subject (including the human), possibly compared to another non-tumoral ("healthy") tissue or another sample (subject cell sample or subject biological sample) from the same subject (which means another tissue, cell or biological fluid of the same subject, but not affected by the tumor disease or disorder). In certain embodiments, the biological sample can also be a germinal cell or a mixture of germinal cells, but preferably can be a somatic cell or a mixture of somatic cells and may also comprise the step of an identification of the sex of the subject (including the human, but preferably not made upon a human embryo or human fetus).

Advancing to a state 230, the method 200 performs an analytical technique upon the biological sample. In some embodiments, the analytical technique may be performed on an automated device for determining genomic, transcriptogenic or epigenomic characteristics. The automated device may be distinct from or part of system 100 shown in Figure 1A or system 100' shown in Figure 1B. The date on which the analytical technique is performed is preferably recorded by the system 100. In certain embodiments, the analytical technique can determine identities of characteristics in the subject's genome, transcriptome and/or epigenome. In some embodiments every genetic characteristic present in this sample genome, this sample transcriptome or this sample epigenome can be compared with more than 10, 20, 30, 40, 50, 100, 500 or more specific genome, transcriptome and/or epigenome characteristics or combination of characteristics (such as, but not limited to elevated or decreased expression levels of one or more genetic sequences) possibly associated with at least one genetic related disorder or disease or possibly associated with an increased risk of a genetic related disorder or disease, these more than 10, 20, 30, 40, 50, 100, 500 or more specific genome, transcriptome and/or epigenome characteristics being stored in a database. This analysis is also advantageously correlated with a possible mode of treatment of (or selection of the most suitable drug or pro-drug to be administrated to) the tested subject belonging to a specific patient to be treated (or treated in prevention of a future disease or disorder). This analysis may also be correlated to the identification of possible valid IP rights (including corresponding royalties or fees dues) protecting this diagnostic or future treatment and will modify in the claimed method the amount of fees charged for the determination of these characteristics as described above... This comparison facilitates an assessment of whether the subject has one or more (two or more, preferably ten or more) characteristics associated with a genetic related disorder or disease or an increased risk of a (genetic related) disorder or disease. In some embodiments, positive results are recorded in an electronic medical record (EMR). In certain embodiments, the characteristics are stored in a centralized genetic database. Specific steps of the method can be obtained by methods and means well known by the person skilled in the art to allow a complete partial or targeted sequencing of different genetic elements, such as the genome, transcriptome or the epigenome of the biological subject comprising a genetic material through known techniques, preferably selected from the group consisting of genetic, transcriptogenic or epigenetic analyses, such as SNP genotyping, detection of sequence methylation, mapping, sequencing, use of high density or low density microarrays, pyrosequencing, gene expression analysis or quantitative genetic amplification and/or DNA fingerprinting.

Proceeding to a state 240, the method 200 determines the identities of a predetermined number of characteristics in the subject's genome, transcriptome and/or epigenome which are associated with one or more particular phenotypes or an increased risk of a particular phenotype. In some embodiments, the genome, transcriptome and/or epigenome characteristics can be a cancer genome, transcriptome and/or epigenome characteristics specific of cancer or tumoral diseases or hyper proliferative disorders. These characteristics could correspond to a benign or malignant cancer disorder of the subject or a disorder susceptible to affect the subject in the future (in a delay of about a few days, a few weeks, about a few months, about one year, about two years, about three years, about four years, about five years, about six years, about seven years, about nine years, about ten years, about fifteen years, about twenty years, about twenty five years, about thirty years, about thirty five years, about forty years, about forty five years, about fifty years, about sixty years, about seventy years, about seventy five years or more). In the method, the genome, transcriptome and/or epigenome characteristic can also be a characteristic selected from the group consisting of diabetes, auto-immune, hypertension, obesity, nerve disease or neurological genome and/or epigenome characteristic, preferably Alzheimer's disease, Parkinson's disease and/or Huntington's disease. In the method, this characteristic can also be selected from the group consisting of Angelman syndrome, Canavan disease, Coeliac disease, Charcot-Marie-Tooth disease, Color blindness disease, Cri du chat disease, Cystic fibrosis disease, Down syndrome, Duchenne muscular dystrophy, Haemochromatosis, Haemophilia, Klinefelter's syndrome, Neurofibromatosis, Phenylketonuria, Polycystic kidney disease, Prader-Willi syndrome, Sickle-cell disease, Tay-Sachs disease, Turner syndrome. The state could be performed upon a multigenic variation, or multi-epigenic variation, genetic modification or monogenic genome and/or epigenome characteristic genetic modification.

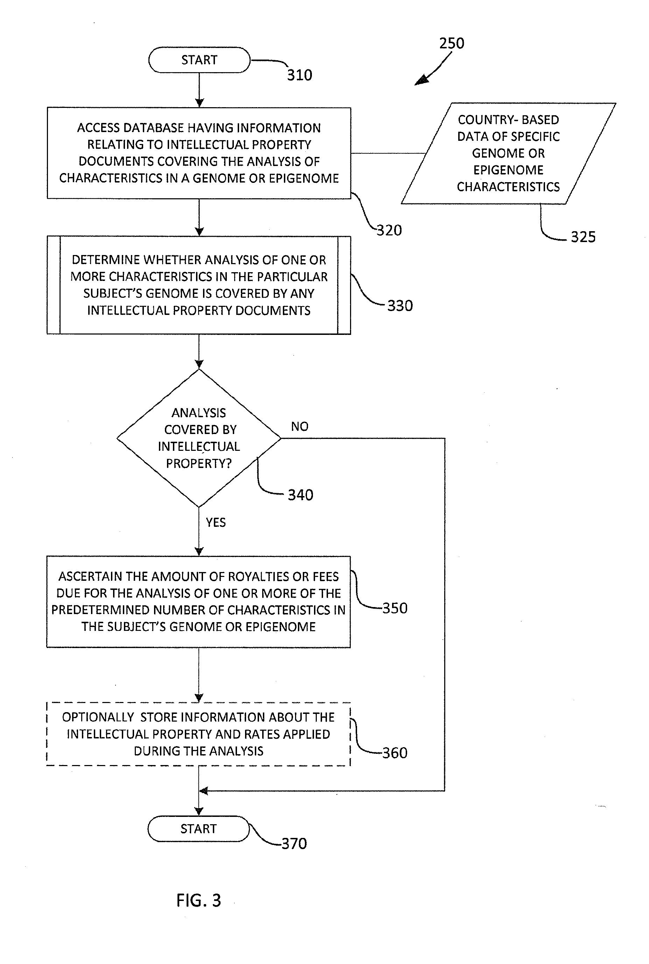

Advancing to a method function 250 or a module, method 200 ascertains an amount of royalties or fees in determining the identities of a predetermined number of characteristics in the subject's genome, transcriptome and/or epigenome. Method function 250 will be further described in conjunction with Figure 3 below. Upon completion of the method function 250, method 200 proceeds to state 260 and adjusts the amount of a fee charged for determining the identities of the predetermined number of characteristics in the particular subject's genome, transcriptome and/or epigenome to reflect the amount of the ascertained royalties or fees and/or an amount of a fee for the future treatment of the subject. In certain embodiments, state 260 can be optional in that the amount of fees is not calculated. In certain embodiments, the different steps of the method can be optionally repeated upon the same particular subject at an interval time comprised between about a few days, about a few weeks or about a few months, preferably after about one year and about ten years, and preferably between about two years and about five years. Process 200 then completes at an end state 270.

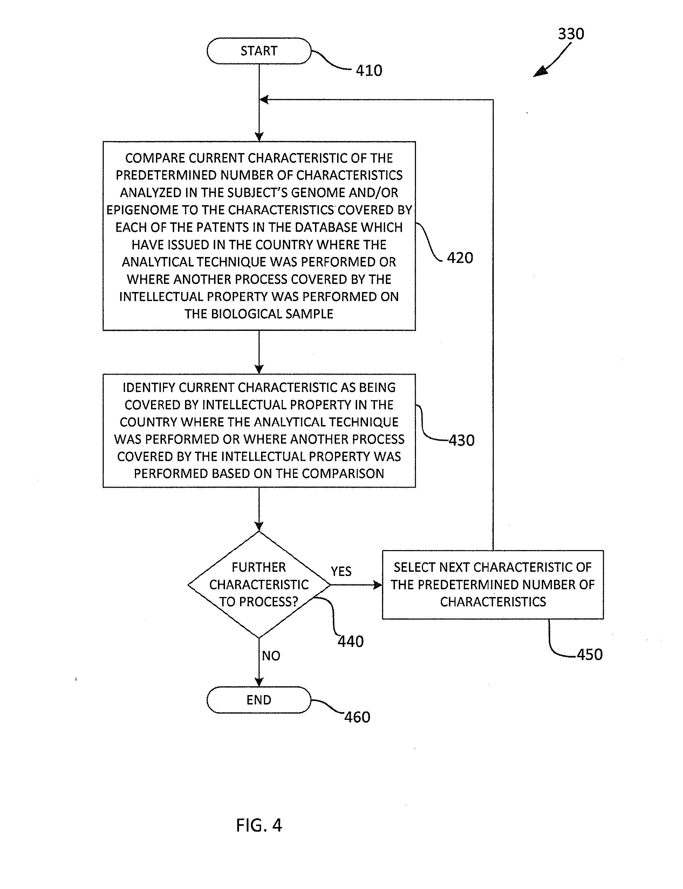

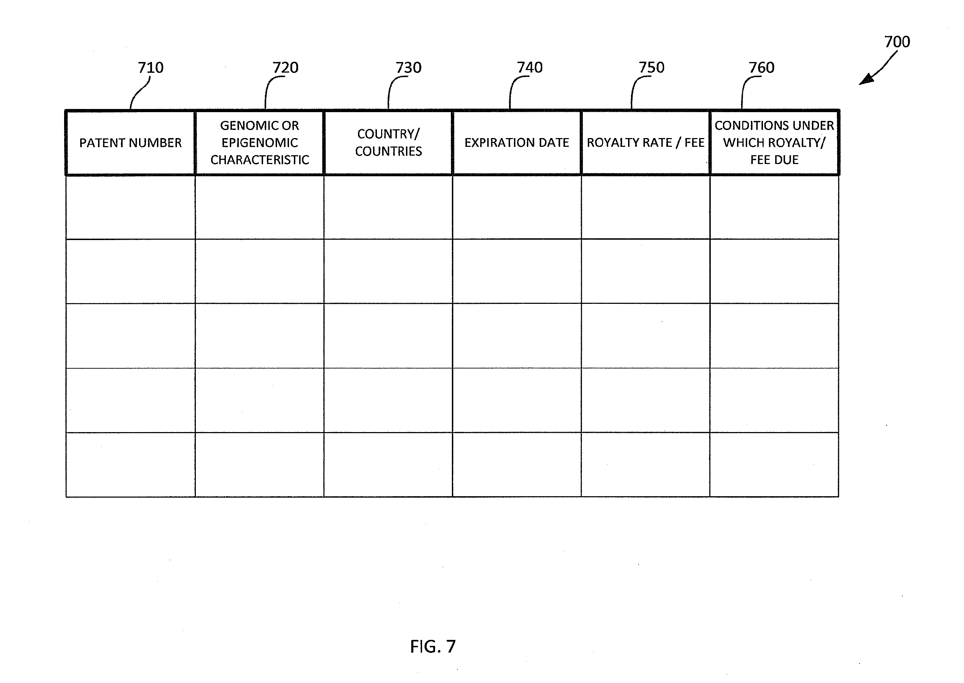

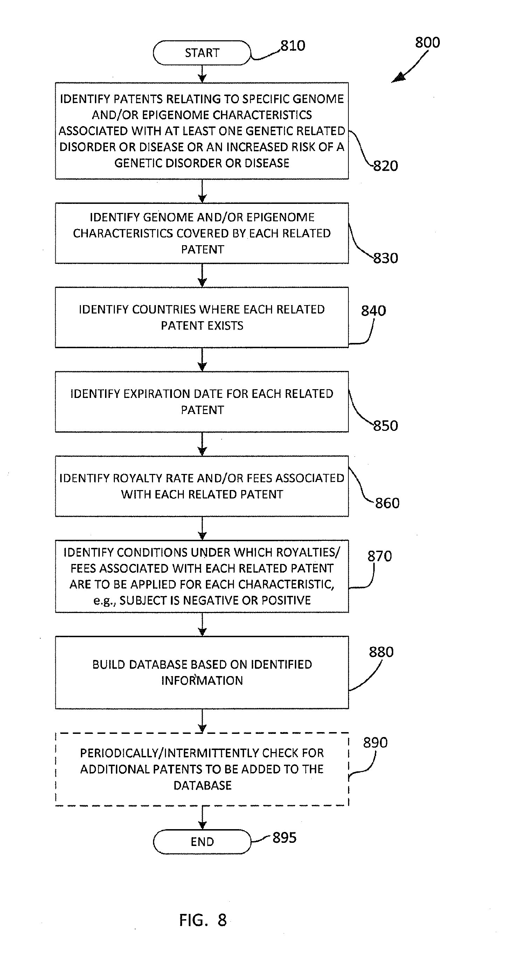

Referring to Figure 3, an example of an embodiment of the method function 250 that can ascertain royalties or fees as shown in Figure 2 will be described. Depending on the embodiment, certain steps of the method function may be added, rearranged, combined, or removed. Beginning at a start state 310, method function 250 proceeds to a state 320 and accesses a database having information relating to intellectual property documents covering the analysis of characteristics in a genome, transcriptome or epigenome. Access can be made to country based data of specific genome, transcriptome or epigenome characteristics 325, which, in certain embodiments, can be derived from a database such as described in conjunction with Figures 7 and 8 herein below. Proceeding to a method function 330, method function 250 determines whether analysis of one or more characteristics in the particular subject's genome, transcriptome or epigenome is covered by any of the intellectual property documents listed in the database. Method function 330 will be further described in conjunction with Figures 4, 5 and 6 below. Upon completion of the method function 330, method function 250 proceeds to a decision state 340 to identify if the analysis is covered by intellectual property documents (i.e. patents or patent applications) listed in the database. If not, method function 250 completes at an end state 370. However, if the analysis is covered by any of the intellectual property documents (patents or patent applications) listed in the database, method function 250 continues at state 350 and ascertains the amount of royalties or fees due for the analysis of one or more of the predetermined number of characteristics in the subject's genome, transcriptome or epigenome. An optional state 360 stores information about the intellectual property applied during the analysis and the rates applied for later reference. Method function 250 then completes at the end state 370.

Referring to Figure 4, an example of an embodiment of the method function 330 that can determine whether analysis of characteristics is covered by intellectual property as shown in Figure 3 will be described. Depending on the embodiment, certain steps of the method function may be added, rearranged, combined, or removed. Beginning at a start state 410, method function 330 proceeds to a state 420 and compares a current characteristic from the predetermined number of characteristics analyzed in the subject's genome, transcriptome and/or epigenome to the characteristics covered by each of the patents in the database which have issued in the country where the analytical technique is performed on the biological sample or where another process covered by the intellectual property is performed on the biological sample. Proceeding to state 430, based on the results of the comparison of state 420, method function 330 identifies the current characteristic as being covered by the intellectual property in the country where the analytical technique was performed or where another process covered by the intellectual property was performed. Advancing to a decision state 440, method function 330 determines whether there is a further characteristic from the predetermined number of characteristics to be processed. If so, method function 330 advances to state 450 and selects a next characteristic from the predetermined number of characteristics. Method function 330 then continues at state 420 with the next characteristic from state 450 as being the current characteristic for processing. However, if there are no further characteristics to process, as determined at decision state 440, method function 330 completes at end state 460.

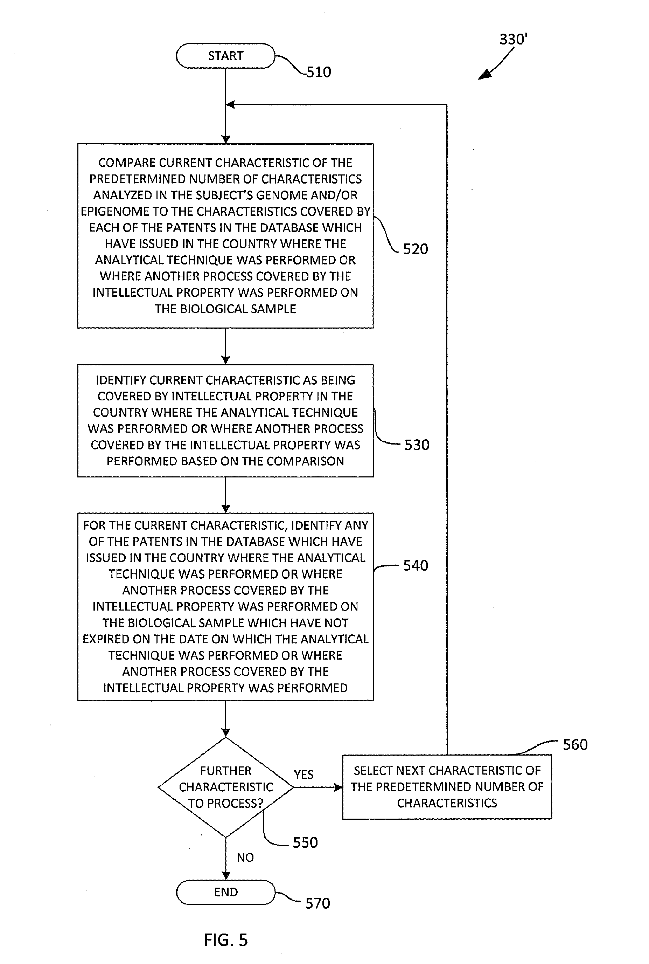

Referring to Figure 5, another example of an embodiment of the method function 330' that can determine whether analysis of characteristics is covered by intellectual property as shown in Figure 3 will be described. Depending on the embodiment, certain steps of the method function may be added, rearranged, combined, or removed. Beginning at a start state 510, method function 330' proceeds to a state 520 and compares a current characteristic from the predetermined number of characteristics analyzed in the subject's genome, transcriptome and/or epigenome to the characteristics covered by each of the patents in the database which have issued in the country where the analytical technique is performed on the biological sample or where another process covered by the intellectual property is performed on the biological sample. Proceeding to state 530, based on the results of the comparison of state 520, method function 330' identifies the current characteristic as being covered by the intellectual property in the country where the analytical technique was performed or where another process covered by the intellectual property was performed. Advancing to state 540, method function 330' identifies for the current characteristic any of the patents in the database which have issued in the country where the analytical technique was performed on the biological sample or where another process covered by the intellectual property was performed on the biological sample which have not expired on the date on which the analytical technique was performed or where another process covered by the intellectual property was performed. Advancing to a decision state 550, method function 330' determines whether there is a further characteristic from the predetermined number of characteristics to be processed. If so, method function 330' advances to state 560 and selects a next characteristic from the predetermined number of characteristics. Method function 330' then continues at state 520 with the next characteristic from state 560 as being the current characteristic for processing. However, if there are no further characteristics to process, as determined at decision state 550, method function 330' completes at end state 570.

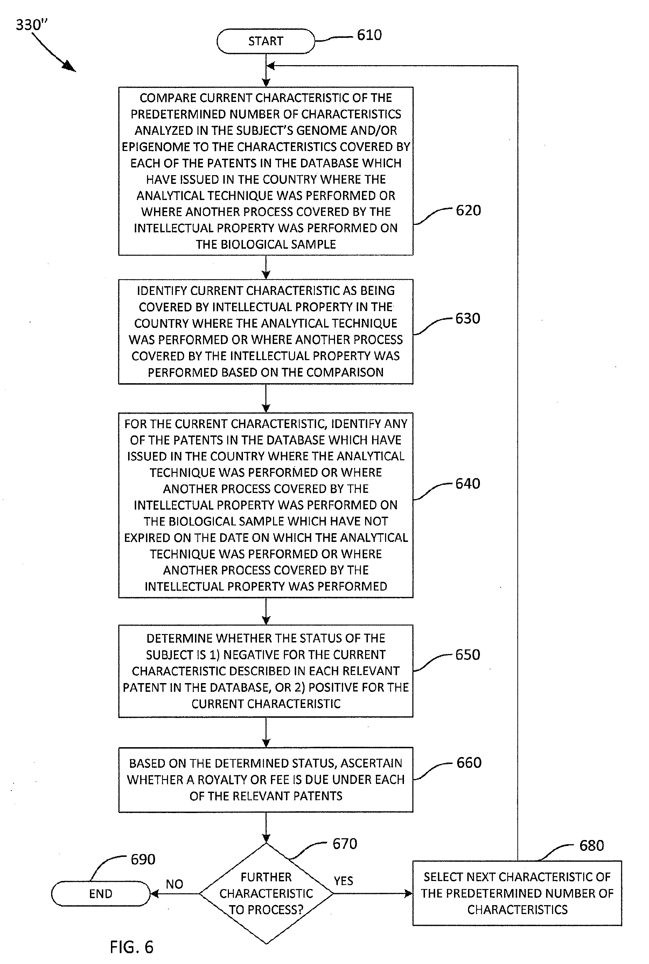

Referring to Figure 6, another example of an embodiment of the method function 330" that can determine whether analysis of characteristics is covered by intellectual property as shown in Figure 3 will be described. Depending on the embodiment, certain steps of the method function may be added, rearranged, combined, or removed. Beginning at a start state 610, method function 330" proceeds to a state 620 and compares a current characteristic from the predetermined number of characteristics analyzed in the subject's genome, transcriptome and/or epigenome to the characteristics covered by each of the patents in the database which have issued in the country where the analytical technique is performed on the biological sample or where another process covered by the intellectual property is performed on the biological sample. Proceeding to state 630, based on the results of the comparison of state 620, method function 330" identifies the current characteristic as being covered by the intellectual property in the country where the analytical technique was performed or where another process covered by the intellectual property was performed. Advancing to state 640, method function 330" identifies for the current characteristic any of the patents in the database which have issued in the country where the analytical technique was performed on the biological sample or where another process covered by the intellectual property was performed which have not expired on the date on which the analytical technique was performed or where another process covered by the intellectual property was performed. Continuing to state 650, method function 330" determines whether the status of the subject is 1) negative for the current characteristic described in each relevant patent in the database, or 2) positive for the current characteristic. Moving to state 660, method function 330" ascertains whether a royalty or fee is due under each of the relevant patents based on the status determined at state 650. For example, for each characteristic, the intellectual property relevant to that characteristic may be assessed to determine whether a royalty or fee is due only if the subject is positive, only if the subject is negative, regardless of whether the subject is positive or negative, or only if the subject has a particular version of that characteristic, such as a methylated nucleotide, an unmethylated nucleotide or a specific nucleotide at a polymorphic site. The conditions under which a royalty or fee is due are dependent on the claims of the relevant intellectual property. Some claims may encompass determining the status of the characteristic only if the status is positive, only if the status is negative or regardless of whether the status is positive or negative. Advancing to a decision state 670, method function 330" determines whether there is a further characteristic from the predetermined number of characteristics to be processed. If so, method function 330' advances to state 680 and selects a next characteristic from the predetermined number of characteristics. Method function 330" then continues at state 620 with the next characteristic from state 680 as being the current characteristic for processing. However, if there are no further characteristics to process, as determined at decision state 670, method function 330" completes at end state 690.

Referring to Figure 7 an example of an embodiment of an intellectual property database 700 accessed during the method of Figure 2 will be described. In certain embodiments, the database 700 includes several fields as follows: a patent number field 710, a genomic and/or epigenomic characteristic field 720 for the characteristic covered by the patent, a country or countries field 730 for where the patent is valid, an expiration date field 740 for when the patent expires, a royalty rate or fee field 750 associated with the covered characteristic, and a conditions field 760 identifying the conditions under which the royalty or fee is due. In certain embodiments, the conditions in field 760 can be that the subject is negative, the subject is positive, or the subject is either negative or positive for the characteristic. A particular patent number may cover more than one characteristic, each of which could have a different associated royalty rate or fee, and/or conditions under which the royalty is due. The fields can be any particular order. In other embodiments, other fields can be added to facilitate execution of the method.

Referring to Figure 8, an example of an embodiment of a method 800 to build an intellectual property database such as shown in Figure 7 will be described. Depending on the embodiment, certain steps of the method may be added, rearranged, combined, or removed. Beginning at a start state 810, method 800 proceeds to state 820 and identifies patents related to specific genome, transcriptome and/or epigenome characteristics associated with at least one genetic related disorder or disease or an increased risk of a genetic disorder or disease. Moving to state 830, method 800 identifies genome, transcriptome and/or epigenome characteristics covered by each related patent. Continuing at state 840 the country or countries where each of the related patents are valid are identified. Advancing to state 850, method 800 identifies an expiration date for each related patent. Proceeding to state 860, method 800 identifies a royalty rate and/or fees associated with each related patent. Moving to state 870, method 800 identifies conditions under which the royalties and/or fees associated with each of the related patents are to be applied for each characteristic. These conditions can be, for example, whether the subject is negative or positive for a particular characteristic. Based on the above information and information for any additional genetic related disorders or diseases affecting or susceptible to affect a subject, a database is built at state 880 using techniques well known in the art. Optionally, method 800 checks for additional patents and/or published applications to be added to the database at a later time at state 890. State 890 can be performed on a periodic or intermittent basis. Method 800 completes at an end state 895.

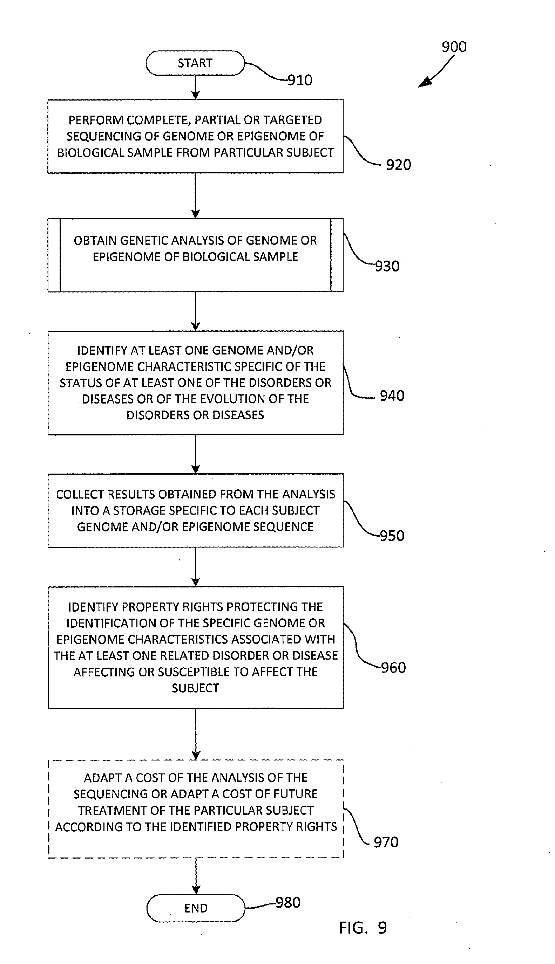

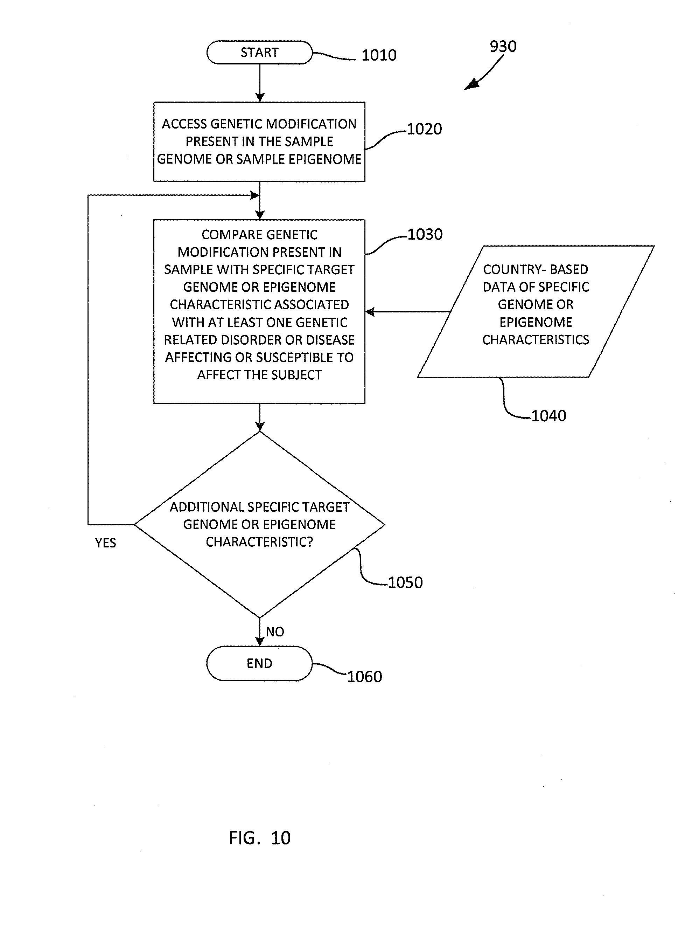

Referring to Figure 9, another example of an embodiment of a method 900 operating on the system shown in Figures 1A or 1B will be described. Depending on the embodiment, certain steps of the method may be added, rearranged, combined, or removed. Beginning at a start state 910, method 900 proceeds to state 920 to perform a complete, partial or targeted sequencing of a genome, transcriptome or epigenome of a biological sample from a particular subject. Continuing at a method function 930, method 900 obtains a genetic analysis of the genome, transcriptome or epigenome of the biological sample. Method function 930 will be described in more detail in conjunction with Figure 10 below. Advancing to state 940, method 900 identifies at least one genome, transcriptome and/or epigenome characteristic specific of the status of at least one of the disorders or diseases or of the evolution of the disorders or diseases. Proceeding to state 950, method 900 collects results obtained from the analysis into a storage specific to each subject genome and/or epigenome sequence. Moving to state 960, process 900 identifies property rights (patents) protecting the identification of the specific genome, transcriptome or epigenome characteristics associated with the at least one related disorder or disease affecting or susceptible to affect the subject. Process 900 then optionally adapts a cost of the analysis of the sequencing or adapts a cost of future treatment of the particular subject according to the identified property rights.

Referring to Figure 10, an example of an embodiment of method function 930 to obtain a genetic analysis as shown in Figure 9 will be described. Depending on the embodiment, certain steps of the method function may be added, rearranged, combined, or removed. Beginning at a start state 1010, method function 930 proceeds to state 1020 to access the genetic modification present in the sample genome, sample transcriptome or sample epigenome. Advancing to state 1030, method function 930 compares the genetic modification present in the sample with a specific target genome, transcriptome or epigenome characteristic associated with at least one genetic related disorder or disease affecting or susceptible to affect the subject. During state 1030, method function 1030 accesses country-based data of specific genome, transcriptome or epigenome characteristics 1040, such as from the database 700 described above. Continuing at a decision state 1050, method function 930 determines if there is an additional specific target genome, transcriptome or epigenome characteristic to be processed. If so, method function 930 continues to state 1030 to process the new specific target genome, transcriptome or epigenome characteristic. However, if method function 930 determines at decision state 1050 that there are no additional specific target genome, transcriptome or epigenome characteristics to be processed, process function 930 completes at an end state 1060.

Various illustrative logics, logical blocks, modules, circuits and algorithm steps described in connection with the implementations disclosed herein may be implemented as electronic hardware, computer software, or combinations of both. The interchangeability of hardware and software has been described generally, in terms of functionality, and illustrated in the various illustrative components, blocks, modules, circuits and steps described above. Whether such functionality is implemented in hardware or software depends upon the particular application and design constraints imposed on the overall system.

In one or more aspects, the functions described may be implemented in hardware, digital electronic circuitry, computer software, firmware, including the structures disclosed in this specification and their structural equivalents thereof, or in any combination thereof. Implementations of the subject matter described in this specification also can be implemented as one or more computer programs, e.g., one or more modules of computer program instructions, encoded on a computer storage media for execution by, or to control the operation of, data processing apparatus.

If implemented in software, the functions may be stored on or transmitted over as one or more instructions or code on a computer-readable medium. The steps of a method or algorithm disclosed herein may be implemented in a processor-executable software module which may reside on a computer-readable medium. Computer-readable media includes both computer storage media and communication media including any medium that can be enabled to transfer a computer program from one place to another. A storage media may be any available media that may be accessed by a computer. By way of example, and not limitation, such computer-readable media may include RAM, ROM, EEPROM, CD-ROM or other optical disk storage, magnetic disk storage or other magnetic storage devices, or any other medium that may be used to store desired program code in the form of instructions or data structures and that may be accessed by a computer. Also, any connection can be properly termed a computer-readable medium. Disk and disc, as used herein, includes compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and instructions on a machine readable medium and computer-readable medium, which may be incorporated into a computer program product.

Certain features that are described in this specification in the context of separate implementations also can be implemented in combination in a single implementation. Conversely, various features that are described in the context of a single implementation also can be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products.

Instructions refer to computer-implemented steps for processing information in the system. Instructions can be implemented in software, firmware or hardware and include any type of programmed step undertaken by components of the system.

Memory refers to electronic circuitry that allows information, typically computer data, to be stored and retrieved. Memory can refer to external devices or systems, for example, disk drives or tape drives. Memory can also refer to fast semiconductor storage (chips), for example, Random Access Memory (RAM) or various forms of Read Only Memory (ROM), which are directly connected to the processor. Other types of memory include bubble memory and core memory.

As can be appreciated by one of ordinary skill in the art, each of the modules of the invention may comprise various sub-routines, procedures, definitional statements, and macros. Each of the modules are typically separately compiled and linked into a single executable program. Therefore, the following description of each of the modules is used for convenience to describe the functionality of the system. Thus, the processes that are undergone by each of the modules may be arbitrarily redistributed to one of the other modules, combined together in a single module, or made available in a shareable dynamic link library. Further each of the modules could be implemented in hardware.

The networks may include any type of electronically connected group of computers including, for instance, the following networks: Internet, Intranet, Local Area Networks (LAN) or Wide Area Networks (WAN). In addition, the connectivity to the network may be, for example, remote modem, Ethernet (IEEE 802.3), Token Ring (IEEE 802.5), Fiber Distributed Datalink Interface (FDDI) or Asynchronous Transfer Mode (ATM). Note that computing devices may be desktop, server, portable, hand-held, set-top, or any other desired type of configuration. As used herein, the network includes network variations such as the public Internet, a private network within the Internet, a secure network within the Internet, a private network, a public network, a value-added network, an intranet, and the like.

An example of one embodiment of the present methods is provided below.

### Example 1

A biological sample is obtained from a test subject and a targeted sequencing analysis is performed with respect to single nucleotide polymorphisms (SNPs) in genes A-J which are associated with an increased risk of diseases A'-J'.

Patents Q-Z contain claims relevant to the analysis of the SNPs in genes A-J respectively and, for each patent, a $20 dollar royalty is due for conducting an analysis covered by the patent. Patents Q-Z have issued in the country where the sequencing analysis is performed. Patents Q-Y have not yet expired on the date on which the sequencing analysis is performed, but Patent Z has expired prior to that date.

Patent Q contains the following claim: "A method for determining whether a subject has an increased risk of suffering from disease A' comprising detecting a G at position 123 of gene A, wherein said subject has an increased risk of suffering from disease A' if said G at position 123 of gene A is present." Since this claim requires detection of a G in the polymorphic position of the SNP, a royalty is only due under Patent Q if a test subject has a G at this position (i.e., a royalty is only due under patent Q if the test subject has a G at position 123 and is positive for an increased risk of disease A').

Patent R contains the following claim: "A method for determining whether a subject has an increased risk of suffering from disease B' comprising determining the identity of the nucleotide at position 123 of gene B, wherein said subject has an increased risk of suffering from disease B' if said polymorphic nucleotide is a C." Since this claim encompasses the detection of any nucleotide at the polymorphic position, a royalty is due under Patent R regardless of whether the test subject is positive or negative and regardless of the identity of the nucleotide at position 123.

Patent S contains the following claim: "A method for determining whether a subject does not have an increased risk of suffering from disease C' comprising detecting an A at position 123 of gene C wherein said subject does not have an increased risk of suffering from disease C' if said A at position 123 of gene C is present." Since this claim requires detection of an A in the polymorphic position of the SNP, a royalty is only due under Patent S if a test subject has an A at this position (i.e., a royalty is only due under patent S if the test subject has an A at position 123 and is negative for an increased risk of disease C').

Patent T contains the following claim: "A method for determining whether a subject has an increased risk of suffering from disease D' comprising determining the identity of the nucleotide at position 123 of gene D, wherein said subject has an increased risk of suffering from disease D' if said polymorphic nucleotide is a C." Since this claim encompasses the detection of any nucleotide at the polymorphic position, a royalty is due under Patent T regardless of whether the test subject is positive or negative and regardless of the identity of the nucleotide at position 123.

Patent U contains the following claim: "A method for determining whether a subject has an increased risk of suffering from disease E' comprising determining the identity of the nucleotide at position 123 of gene E, wherein said subject has an increased risk of suffering from disease E' if said polymorphic nucleotide is a C." Since this claim encompasses the detection of any nucleotide at the polymorphic position, a royalty is due under Patent U regardless of whether the test subject is positive or negative and regardless of the identity of the nucleotide at position 123.

Patent V contains the following claim: "A method for determining whether a subject has an increased risk of suffering from disease F' comprising determining the identity of the nucleotide at position 123 of gene F, wherein said subject has an increased risk of suffering from disease F' if said polymorphic nucleotide is a C." Since this claim encompasses the detection of any nucleotide at the polymorphic position, a royalty is due under Patent V regardless of whether the test subject is positive or negative and regardless of the identity of the nucleotide at position 123.

Patent W contains the following claim: "A method for determining whether a subject has an increased risk of suffering from disease G' comprising determining the identity of the nucleotide at position 123 of gene G, wherein said subject has an increased risk of suffering from disease G' if said polymorphic nucleotide is a C." Since this claim encompasses the detection of any nucleotide at the polymorphic position, a royalty is due under Patent W regardless of whether the test subject is positive or negative and regardless of the identity of the nucleotide at position 123.

Patent X contains the following claim: "A method for determining whether a subject has an increased risk of suffering from disease H' comprising determining the identity of the nucleotide at position 123 of gene H, wherein said subject has an increased risk of suffering from disease H' if said polymorphic nucleotide is a C." Since this claim encompasses the detection of any nucleotide at the polymorphic position, a royalty is due under Patent X regardless of whether the test subject is positive or negative and regardless of the identity of the nucleotide at position 123.

Patent Y contains the following claim: "A method for determining whether a subject has an increased risk of suffering from disease I' comprising determining the identity of the nucleotide at position 123 of gene I, wherein said subject has an increased risk of suffering from disease I' if said polymorphic nucleotide is a C." Since this claim encompasses the detection of any nucleotide at the polymorphic position, a royalty is due under Patent Y regardless of whether the test subject is positive or negative and regardless of the identity of the nucleotide at position 123.

Patent Z contains the following claim: "A method for determining whether a subject has an increased risk of suffering from disease J' comprising determining the identity of the nucleotide at position 123 of gene J, wherein said subject has an increased risk of suffering from disease J' if said polymorphic nucleotide is a C." Since this claim encompasses the detection of any nucleotide at the polymorphic position, a royalty would be due under Patent Z regardless of whether the test subject is positive or negative and regardless of the identity of the nucleotide at position 123. However, as noted above, Patent Z has expired prior to the date on which the sequencing analysis is performed.

Information regarding Patents Q-Z is included in a database having the format and information provided in Figure 7. In certain embodiments, the database is stored in a computing device. The computing device may be part of a system such as the systems illustrated in Figure 1A or Figure 1B.

The targeted sequencing analysis reveals that the test subject has a G at position 123 of gene A. As noted above, Patent Q relates to the analysis of position 123 of gene A and a royalty is due only if the test subject has a G at that position. The results of the sequencing analysis on the test subject are input into the computing device or the system and the information from the test subject is compared to the conditions under which a royalty is required under patent Q. Since the test subject is positive and since a royalty is due under Patent Q when the test subject is positive, a $20 royalty is due under Patent Q.

The targeted sequencing analysis reveals that the test subject has a C at position 123 of gene B. As noted above, Patent R relates to the analysis of position 123 of gene B and a royalty is due regardless of whether the test subject is positive or negative. The results of the sequencing analysis on the test subject are input into the computing device or the system and the information from the test subject is compared to the conditions under which a royalty is required under patent R. Since the test subject is positive and since a royalty is due under Patent Q when the test subject is positive or negative, a $20 royalty is due under Patent R.

The targeted sequencing analysis reveals that the test subject has a C at position 123 of gene C. As noted above, Patent S relates to the analysis of position 123 of gene C and a royalty is due only when the test subject has an A at this position. The results of the sequencing analysis on the test subject are input into the computing device or the system and the information from the test subject is compared to the conditions under which a royalty is required under patent S. Since the test subject has a C at position 123 of gene C and since a royalty is only due under Patent S when the test subject has an A at this position, no royalty is due under Patent S.

The targeted sequencing analysis reveals that the test subject has a G at position 123 of gene D. As noted above, Patent T relates to the analysis of position 123 of gene D and a royalty is due regardless of the identity of the nucleotide at this position. The results of the sequencing analysis on the test subject are input into the computing device or the system and the information from the test subject is compared to the conditions under which a royalty is required under patent T. Since a royalty is due under Patent T regardless of the identity of the nucleotide at position 123, a $20 royalty is due under Patent T.

The targeted sequencing analysis reveals that the test subject has a C at position 123 of gene E. As noted above, Patent U relates to the analysis of position 123 of gene E and a royalty is due regardless of the identity of the nucleotide at this position. The results of the sequencing analysis on the test subject are input into the computing device or the system and the information from the test subject is compared to the conditions under which a royalty is required under patent U. Since a royalty is due under Patent U regardless of the identity of the nucleotide at position 123, a $20 royalty is due under Patent U.

The targeted sequencing analysis reveals that the test subject has an A at position 123 of gene F. As noted above, Patent V relates to the analysis of position 123 of gene F and a royalty is due regardless of the identity of the nucleotide at this position. The results of the sequencing analysis on the test subject are input into the computing device or the system and the information from the test subject is compared to the conditions under which a royalty is required under patent V. Since a royalty is due under Patent V regardless of the identity of the nucleotide at position 123, a $20 royalty is due under Patent V.

The targeted sequencing analysis reveals that the test subject has a T at position 123 of gene G. As noted above, Patent W relates to the analysis of position 123 of gene G and a royalty is due regardless of the identity of the nucleotide at this position. The results of the sequencing analysis on the test subject are input into the computing device or the system and the information from the test subject is compared to the conditions under which a royalty is required under patent W. Since a royalty is due under Patent W regardless of the identity of the nucleotide at position 123, a $20 royalty is due under Patent W.

The targeted sequencing analysis reveals that the test subject has an A at position 123 of gene H. As noted above, Patent X relates to the analysis of position 123 of gene H and a royalty is due regardless of the identity of the nucleotide at this position. The results of the sequencing analysis on the test subject are input into the computing device or the system and the information from the test subject is compared to the conditions under which a royalty is required under patent X. Since a royalty is due under Patent X regardless of the identity of the nucleotide at position 123, a $20 royalty is due under Patent X.

The targeted sequencing analysis reveals that the test subject has an A at position 123 of gene I. As noted above, Patent Y relates to the analysis of position 123 of gene I and a royalty is due regardless of the identity of the nucleotide at this position. The results of the sequencing analysis on the test subject are input into the computing device or the system and the information from the test subject is compared to the conditions under which a royalty is required under patent Y. Since a royalty is due under Patent Y regardless of the identity of the nucleotide at position 123, a $20 royalty is due under Patent Y.

The targeted sequencing analysis reveals that the test subject has an A at position 123 of gene J. As noted above, Patent Z relates to the analysis of position 123 of gene J and a royalty would be due regardless of the identity of the nucleotide at this position. However, Patent Z has expired prior to the date on which the sequencing analysis was performed. Therefore, no royalty is due under Patent Z.

In view of the foregoing example results of the sequencing analysis, $160 in royalties is due for the analysis of this specific test subject. Accordingly, $160 is added to the invoice for conducting the analysis of the specific test subject to reflect the royalties that had to be paid to conduct the analysis.

### Conclusion

The foregoing description details certain embodiments.that can be combined in the method and system according to the invention to identify various IP rights (Patents and collection of Data bases) to be respected and from which royalties and fees are due. It will be appreciated, however, that no matter how detailed the foregoing appears in text, the invention may be practiced in many ways. It should be noted that the use of particular terminology when describing certain features or aspects of the invention should not be taken to imply that the terminology is being re-defined herein to be restricted to including any specific characteristics of the features or aspects of the invention with which that terminology is associated.

While the above detailed description has shown, described, and pointed out novel features of the invention as applied to various embodiments, it will be understood that various omissions, substitutions, and changes in the form and details of the device or process illustrated may be made by those skilled in the technology without departing from the intent of the invention. The scope of the invention is indicated by the appended claims rather than by the foregoing description. All changes which come within the meaning and range of equivalency of the claims are to be embraced within their scope.

## Claims

1. A method for performing an analysis of a subject's (preferably a human's) genome, transcriptome and/or epigenome, the method comprising:
determining the identities of two or or more, preferably ten or more characteristics in said subject's (preferably a human individual's) genome, transcriptome and/or epigenome which are associated with one or more particular phenotype(s) or with an increased risk of a particular phenotype by performing an analytical technique upon a biological sample obtained from said subject (preferably said human individual) ;
possibly recording these identities upon the electronic medical recorder (EMR) specific of the subject, preferably specific of the human individual;
determining, via a computer, the amounts of any royalties or fees required for determination of the identities of one or more of said two or more, preferably ten or more characteristics in the subject (human individual )'s genome, transcriptome and/or epigenome; and
adjusting, via a computer, the amount of a fee charged for a determination of the said identities of said two or more, preferably ten or more characteristics in the subject (human individual)'s genome, transcriptome and/or epigenome, to reflect the amount of the determined royalties or fees and/or the amount of a fee for the future treatment of said subject, preferably said human individual.

2. The method of Claim 1, wherein said ten or more characteristics are selected from the group consisting of an allele of a gene, a polymorphic nucleotide, a deletion of one or more nucleotides, an insertion of one or more nucleotides, a translocation of one or more nucleotides, an inversion of one or more nucleotides, a duplication or triplication of one or more nucleotides, a sequence copy number variation a transcript expression level of a nucleic acid sequence or portion thereof, and a methylation status of one or more nucleotides.

3. The method of Claim 1 or 2, wherein determining the amounts of any royalties or fees required for determination of the identities of one or more of said ten or more characteristics comprises:
instructing a computer to access a database having stored therein information relating to a plurality of intellectual property documents covering the analysis of characteristics in a genome, transcriptome and/or epigenome;
instructing a computer to determine whether the analysis of one or more of said ten or more characteristics in the subject (human individual)'s genome, transcriptome and/or epigenome is covered by any of said intellectual property documents (preferably patents); and
if the analysis of one or more of said ten or more characteristics in the subject's genome, transcriptome and/or epigenome is covered by said intellectual property, instructing a computer to determine the amount of the royalties or fees due for said analysis of one or more of said ten or more characteristics in the subject (human individual)'s genome, transcriptome and/or epigenome.

4. The method of Claim 3, wherein the database comprises information relating to the characteristics covered by each of said documents (patents), the countries in which each of said documents (patents) are issued, and the royalty or fee required under each of said documents (patents) in each of said countries and wherein determining whether the analysis of one or more of said ten or more characteristics in the subject (human individual)'s genome, transcriptome and/or epigenome is covered by said intellectual property comprises comparing each of said ten or more characteristics analyzed in said subject (human individual)'s genome, transcriptome and/or epigenome to the characteristics covered by each of said documents (patents) in said database which have issued in the country where said analytical technique was performed on said biological sample to identify any of said ten or more characteristics which are covered by intellectual property in said country, wherein the database further comprises the expiration dates of each of said documents (patents) in each of said countries and wherein determining whether the analysis of one or more of said ten or more characteristics in the subject (human individual)'s genome and/or epigenome is covered by said intellectual property further comprises identifying any of said documents (patents) issued in said country where said analytical technique was performed on said biological sample which had not expired on the date on which said analytical technique was performed and wherein the database further comprises information for each document (patent) relating to whether a royalty or fee is due under said document (patent) if said subject is negative for the characteristic described therein, whether a royalty or fee is due under said document (patent) if said subject is positive for the characteristic described therein, whether a royalty or fee is due under said document (patent) if said subject is either positive or negative for the characteristic described therein, whether a royalty or fee is due only if said characteristic has a specific identity or whether a royalty or fee is due regardless of the identity of said characteristic, and wherein determining whether the analysis of one or more of said ten or more characteristics in the subject (human individual)'s genome, transcriptome and/or epigenome is covered by said intellectual property further comprises comparing the status of each of said ten or more characteristics in said subject, preferably said human individual to the statuses of the characteristics which are royalty or fee bearing under each of said documents (patents).

5. A method for performing an analysis of a subj ect (human individual) 's genome, transcriptome and/or epigenome, the method comprising:
determining the identities of two or more characteristics in said subject (human individual)'s genome, transcriptome and/or epigenome which are associated with one or more particular phenotype(s) or with an increased risk of one or more particular phenotype(s) by performing an analytical technique upon a biological sample obtained from said subject, preferably said human individual;
determining, via a computer, the amounts of any royalties or fees required for determination of the identities of one or more of said two or more characteristics in the subject (human individual)'s genome, transcriptome and/or epigenome; and
adjusting, via a computer, the amount of a fee charged for a determination of the said identities of said two or more characteristics in the subject (human individual)'s genome, transcriptome and/or epigenome, to reflect the amount of the determined royalties or fees and/or the amount of a fee for the future treatment of said subject, preferably said human individual.

6. A computerized system for performing an analysis of a subject (human individual)'s genome, transcriptome and/or epigenome, the system comprising:
a database configured to store information regarding the identities of ten or more characteristics in the subject (human individual)'s genome, transcriptome and/or epigenome which are associated with particular phenotypes or with an increased risk of particular phenotype(s) and information regarding the amounts of any royalties required for determination of the identities of one or more of said ten or more characteristics in the subject (human individual)'s genome, transcriptome and/or epigenome; and
a processor configured to perform instructions for:
determining the amount of any royalties required for determination of the identities of one or more of said ten or more characteristics in the subject (human individual) 's genome, transcriptome and/or epigenome; and
adjusting the amount of a fee charged for said determination of said identities of said ten or more characteristics in the subject (human individual) 's genome, transcriptome and/or epigenome to reflect the amount of the determined royalties.

7. A non-transitory computer readable medium containing program instructions for genetic analysis, wherein execution of the program instructions by a computing environment carries out a method, comprising:
retrieving information regarding identities of ten or more characteristics in a subject (human individual)'s genome, transcriptome and/or epigenome which are associated with particular phenotypes from a database;
retrieving information regarding amounts of any royalties required for determination of said identities of one or more of said ten or more characteristics in the subject (human individual)'s genome, transcriptome and/or epigenome from a database;
determining amounts of any royalties required for determination of the identities of one or more of said ten or more characteristics in the subject (human individual)'s genome, transcriptome and/or epigenome; and
adjusting the amount of a fee charged for said determination of said identities of said ten or more characteristics in the subject (human individual)'s genome, transcriptome and/or epigenome to reflect the amount of the determined royalties.

8. A method to identify genetic modifications in a specific subject suffering or susceptible to suffer from one or more genetic related disorder(s) or disease(s) affecting this specific subject, preferably a specific human individual, the method comprising:
performing a complete, partial or targeted sequencing of the genome, the transcriptome and/or the epigenome of a biological sample obtained from said specific subject, preferably said specific human individual;
obtaining genetic analysis of genome, transcriptome and/or epigenome of the biological sample by comparing every genetic modification present in the said sample genome, said sample transcriptome and/or said sample epigenome with more than ten specific genome, transcriptome and/or epigenome characteristics associated one or more genetic related disorder(s) or disease(s) affecting or susceptible to affect a subject, the said ten or more specific genome, transcriptome and/or epigenome characteristics being present in a centralized database;
identifying at least one genome, transcriptome and/or epigenome characteristic specific of the status of at least one of the said disorders or diseases or of the evolution of the said disorders or diseases;
possibly identifying the sex of the specific subject, preferably the sex of the specific human individual
collecting positive results obtained from said comparison in a database specific for each subject genome, transcriptome and/or epigenome sequenced;
adapting the cost or fee of the analysis of this sequencing or adapting the costs or fees of a future treatment of the said subject, preferably said human individual, according to at least one royalty rate of at least one property right protecting the identification of one or more of the said specific genome, transcriptome and/or epigenome characteristic(s) and
possibly issuing an invoice reflecting the adapted cost.

9. The method according to Claim 8, wherein the genetic analysis is obtained by comparing every genetic modification present in the sample genome, transcriptome or epigenome with 50 or more specific genome, transcriptome and/or epigenome characteristics associated with at least one genetic related disorder(s) or disease(s) affecting the subject, preferably the human individual.

10. The method according to Claim 8 or 9, wherein the genetic analysis is obtained by comparing every genetic modification present in the sample genome, transcriptome or epigenome with 100 or more specific genome, transcriptome and/or epigenome characteristics associated with at least one genetic related disorder(s) or disease(s) affecting the subject, preferably the human individual.

11. The method according to any of the preceding Claims 8 to 10, wherein the genetic analysis is obtained by comparing every genetic modification present in the sample genome, transcriptome or epigenome with 500 or more specific genome, transcriptome and/or epigenome characteristics associated with at least one genetic related disorder(s) or disease(s) affecting the subject, preferably the human individual.

12. The method according to any of the preceding Claims 8 to 11, being repeated upon the same subject, preferably the same human individual at an interval time comprised between one week and ten years.

13. The method according to any of the preceding Claims 8 to 12, wherein the complete, partial or targeted sequencing is based upon methods selected from the group consisting of genetic or epigenetic analyses, SNP genotyping, detection of sequence methylation, mapping, sequencing, use of high density or low density microarrays, pyrosequencing, gene expression analysis, transcript expression or quantitative genetic amplification and/or DNA fingerprinting.

14. The method according to any of the preceding Claims 8 to 13, wherein the comparing is performed upon multigenic variation, genetic modification or monogenic genome, transcriptome and/or epigenome characteristic genetic modification.

15. A method for performing an analysis of a subject (human individual)'s genome, trancriptome and/or epigenome, the method comprising:
determining the identities often or more characteristics in said specific subject (human individual)'s genome, transcriptome and/or epigenome which are associated with one or more particular phenotype(s) or with an increased risk of one or more particular phenotype(s), by performing an analytical technique upon a biological sample obtained from said subject, preferably said human individual, wherein said analytical technique is able to determine identities of the said at least ten or more characteristics of the subject (human individual)'s genome, transcriptome and/or epigenome;
determining the amounts of any royalties required for determination of the identities of one or more of said ten or more characteristics in the subject (human individual)'s genome, transcriptome and/or epigenome; and
adjusting the amount of fee charged for a determination of the said identities of said ten or more characteristics in the subject (human individual)'s genome, transcriptome and/or epigenome, to reflect the amount of the determined royalties and/or the amount of fee for the future treatment of said subject, preferably said human individual.
